Europäisches Patentamt

**European Patent Office**

**Office européen des brevets**

(19)

(11) Numéro de publication : **0 117 818**
**B1**

(12)

# FASCICULE DE BREVET EUROPÉEN

(45) Date de publication du fascicule du brevet :
**23.12.87**

(21) Numéro de dépôt : **84400375.6**

(22) Date de dépôt : **24.02.84**

(51) Int. Cl.⁴ : **A 61 F  5/47**

(54) **Dispositif contraceptif intra-utérin.**

(30) Priorité : **01.03.83 FR 8303358**

(43) Date de publication de la demande :
**05.09.84 Bulletin 84/36**

(45) Mention de la délivrance du brevet :
**23.12.87 Bulletin 87/52**

(84) Etats contractants désignés :
**AT BE CH DE FR GB IT LI LU NL SE**

(56) Documents cités :
**DE-C-  214 172**
**FR-A-  2 427 088**
**US-A-  3 405 711**

(73) Titulaire : **LABORATOIRE CENTRAL DE CHIMIOTHE-**
**RAPIE ET DERMOCHIMIE ( C.C.D.) Société anonyme**
**dite:**
**44, rue Notre -Dame de Lorette**
**F-75009 Paris (FR)**

(72) Inventeur : **Audebert, Alain**
**40 Cours de Verdun**
**F-33000 Bordeaux (FR)**

(74) Mandataire : **Combe, André**
**CABINET BEAU DE LOMENIE 55, rue d'Amsterdam**
**F-75008 Paris (FR)**

Jouve, 18, rue St-Denis, 75001 Paris, France

## Description

La présente invention concerne, en tant que produit industriel nouveau, un dispositif contraceptif intra-utérin.

Des dispositifs de contraception intra-utérins, (désignés ci-après D.I.U. par commodité) sont connus depuis longtemps. Il s'agit essentiellement de dispositifs comprenant une tige et un moyen de retenue dans l'utérus lié à ladite tige, ladite tige et ledit moyen étant, après insertion, tous deux logés à l'intérieur de la cavité utérine. Parmi les D.I.U. connus qui ont été commercialisés, existent des dispositifs bidimensionnels, d'une part, tels que les « 7 » et « T » qui présentent l'inconvénient d'être susceptibles d'être expulsés spontanément, ou encore le dispositif en forme de boucle munie d'éperons ou tentacules s'étendant latéralement et empêchant l'expulsion spontanée, qui est notamment décrit dans FR-A-2 023 276, mais qui présente l'inconvénient d'être susceptible de déchirer la paroi de la cavité utérine ; et des dispositifs tridimensionnels, d'autre part, tels que le dispositif en forme de fleur de lys décrit dans US-A-3 734 089 qui présentent l'inconvénient de soulever des difficultés au niveau de l'insertion, le passage de la structure tridimensionnelle dans le col utérin étant particulièrement délicat.

On peut également citer le brevet FR-A-2 427 088 qui décrit un dispositif contraceptif intra-utérin constitué par une tige et deux paires de bras souples, fixés par l'une de leurs extrémités à la partie supérieure de la tige et destinés à retenir le dispositif dans la cavité utérine. Lors de l'introduction de ce dispositif, les bras sont allongés de part et d'autre de la tige. Au-delà du col utérin, les bras reprennent leur position initiale. De ce fait, les extrémités libres des bras sont susceptibles d'être mobiles le long de la tige.

Selon l'invention, on propose une nouvelle solution technique différente de celles de l'art antérieur et destinée à pallier leurs inconvénients, permettant une insertion puis, le moment venu, un retrait aisés, assurant une bonne tolérance endométriale et une bonne rétention, et empêchant de façon fiable la nidation. Cette nouvelle solution technique fait appel à un dispositif bidimensionnel au niveau de l'insertion et qui présente une structure à dimensions variables par déformation — et éventuellement tridimensionnelle — à l'intérieur de la cavité utérine sous l'influence des mouvements naturels de l'utérus, cette structure tridimensionnelle s'opposant à l'expulsion spontanée.

Le D.I.U. selon l'invention, réalisé en matière plastique, comprend une tige et des moyens souples de retenue formés de deux bras latéraux devant être logés tous deux entièrement dans la cavité utérine, et qui, avant insertion, sont symétriques par rapport à l'axe de la tige et sont situés dans un plan vertical passant par ledit axe, chaque bras latéral étant fixé solidairement par l'une de ses extrémités à la partie supérieure de la tige, ledit dispositif étant caractérisé en ce que chacun desdits bras est lié par son autre extrémité à une bague entourant ladite tige et susceptible de coulisser le long de celle-ci.

Selon une variante de réalisation, les deux bras latéraux sont chacun munis, à leur partie supérieure, d'un prolongement latéral pour contact avec la cavité utérine au voisinage de la lumière de chaque trompe de Fallope et, à leur partie inférieure, d'une aspérité arrondie pour faciliter les contacts avec la paroi de l'utérus.

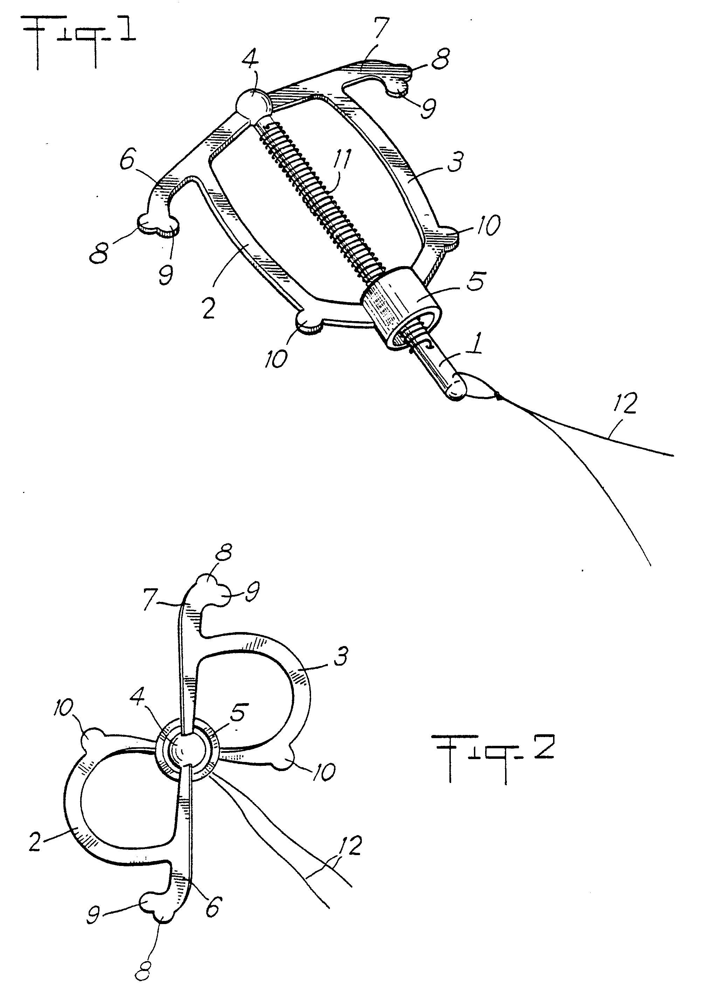

D'autres avantages et caractéristiques de l'invention seront mieux compris à la lecture de la description qui va suivre des dessins annexés sur lesquels la figure 1 représente en perspective un D.I.U. selon l'invention et la figure 2 une vue de dessus de ce D.I.U. après coulissement de la bague vers l'extrémité supérieure de la tige.

Le D.I.U. conçu notamment en polyéthylène ou polypropylène comprend une tige 1 et deux bras latéraux représentés en 2 et 3 et formant chacun une boucle souple. Ces bras latéraux qui avant insertion, sont symétriques par rapport à l'axe de la tige et situés dans un même plan vertical passant dans ledit axe, sont chacun fixés solidairement par l'une de leurs extrémités à la partie supérieure 4 de la tige, et sont liés chacun par la seconde de leurs extrémités à une bague 5 susceptible de coulisser le long de la tige 1. La course maximale de la bague 5 est inférieure à la longueur totale de la tige (qui est ici avantageusement de l'ordre de 31,5 mm).

Avant insertion, chaque bras latéral forme approximativement avec la tige du D.I.U. une courbe de type trapèze curviligne dont la grande base serait constituée par la tige. Le bras 2 est pourvu à sa partie supérieure et plus précisément au niveau du sommet supérieur de sa petite base, d'un prolongement latéral 6 légèrement replié sur lui-même, de même que le bras 3 est également pourvu d'un prolongement latéral 7 identique à celui du bras 2. Les prolongements 6 et 7 sont destinés à venir chacun en contact avec la paroi de la cavité utérine au voisinage de la lumière des trompes de Falloppe. A cet effet, chaque prolongement est muni à son extrémité libre de deux aspérités 8 et 9, pour éviter toute déchirure ou lésion de ladite paroi.

Le sommet inférieur de la petite base de chaque bras latéral est également muni d'une aspérité arrondie 10 pour faciliter les contacts avec la paroi de la cavité utérine sans provoquer de lésions, d'une part, et conférer une certaine résistance mécanique à la boucle souple constituée par ledit bras latéral, d'autre part.

La tige du D.I.U. est pourvue d'un enroulement métallique 11 conçu en cuivre (de préférence) ou en alliage de cuivre, notamment en laiton, pour bénéficier de l'effet contraceptif résultant du délitage du cuivre dans la cavité utérine. La surface de cuivre utile sera avantageusement de l'ordre de 250 mm² à 400 mm² pour assurer une protection efficace de l'ordre de trois à quatre ans. La tige est également liée, au voisinage de son extrémité inférieure, à un fil chirurgical 12 devant être logé dans le col et suturé sur le rebord inférieur de ladite tige.

La figure 2 montre la déformation des bras latéraux qui intervient quand la bague 5 coulisse le long de ladite tige 1. Le mouvement de la bague 5 en translation le long de ladite tige modifie la conformation du D.I.U., le repoussant vers le fond de l'utérus en élargissant et en déformant sa structure et lui permettant de résister aux expulsions au niveau isthmique.

Le D.I.U. selon l'invention est inséré selon une méthode connue en soi au moyen d'un tube creux souple à l'extrémité supérieure duquel est logée la tige, et dans lequel coulisse un organe pousseur. Le D.I.U. selon l'invention est retiré facilement selon une technique classique quand une telle opération est nécessaire.

Les essais cliniques qui ont été entrepris pendant quinze mois sur 400 patientes toutes volontaires âgées de 18 à 35 ans, ont permis de mettre en évidence que le D.I.U. selon l'invention est bien toléré (aucune lésion, aucune expulsion spontanée, aucun retrait jugé nécessaire) et très fiable (aucune grossesse).

**Revendications**

1. Dispositif contraceptif devant être logé à l'intérieur de la cavité utérine, qui comprend une tige (1), et des moyens de retenue formés de deux bras latéraux souples (2) (3) qui, avant sont situés dans un plan passant par ledit axe, chacun desdits bras étant fixés solidairement par l'une de leurs extrémités à la partie supérieure (4) de ladite tige, caractérisé en ce que chacun desdits bras (2) (3) est lié par son autre extrémité à une bague (5) entourant ladite tige et susceptible de coulisser le long de ladite tige.

2. Dispositif selon la revendication 1, caractérisé en ce que lesdits bras latéraux (2) (3) sont chacun munis, à leur partie supérieure, d'un prolongement latéral (6) (7) pour contact avec la cavité utérine au voisinage de la lumière de chaque trompe de Fallope, et à leur partie inférieure, d'une aspérité arrondie (10) pour faciliter les contacts avec la paroi de l'utérus.

**Claims**

1. Contraceptive device adapted to be housed inside the uterine cavity, comprising a stem (1), and two retaining means constituted by two supple side arms (2) (3) which, before the insertion, are symmetrical with respect to the axis of said stem and are situated inside a plane traversing said axis, each arm being firmly connected by one of their ends to the upper part (4) of the stem, characterized in that each of said arms (2) (3) is joined by its second end to a ring member (5) surrounding said stem and capable of sliding along said stem.

2. Device according to claim 1, characterized in that said side arms (2) (3) are each provided, at their upper part, with a side extension (6) (7) designed to come into contact with the uterine cavity close to the lumen of each Fallopian tube, and at the lower part, with a rounded excescence (10) designed to facilitate the contacts with the wall of the uterus.

**Patentansprüche**

1. Kontrazeptive Vorrichtung zum Anbringen im Inneren der Gebärmutterhöhle, umfassend eine Stange (1) und Haltemittel, die durch zwei nachgiebige seitliche Arme (2) (3) gebildet sind, welche vor dem Einbringen symmetrisch zur Achse der Stange sind und in einer durch die Achse gehenden Ebene liegen, wobei jeder Arm an einem seiner Enden mit dem oberen Teil (4) der Stange fest verbunden ist, dadurch gekennzeichnet, daß jeder Arm (2) (3) mit seinem anderen Ende an einem die Stange umgebenden und entlang der Stange gleitbaren Ring (5) befestigt ist.

2. Vorrichtung nach Anspruch 1, dadurch gekennzeichnet, daß die seitlichen Arme (2) (3) jeweils in ihrem oberen Teil mit einer seitlichen Verlängerung (6) (7) für den Kontakt mit der Gebärmutterhöhle in Nähe der Lichtung jeder Fallopio-Tube und in ihrem unteren Teil mit einer abgerundeten Ausbuchtung (10) zur Erleichterung des Kontakts mit der Gebärmutterwand versehen sind.

Fiq.1

Fiq.2